# EUROPEAN PATENT APPLICATION

(11) **EP 0 958 835 A1**
(43) Date of publication of application: **24.11.1999**
(21) Application number: 97934760.6
(22) Date of filing: 11.08.1997
(51) Int. Cl.: A61K 45/00, A61K 31/415

(54) **PREVENTIVE/REMEDY FOR FREQUENT URINATION AND URINARY INCONTINENCE**

(30) Priority: 19.08.1996 JP 25368696
(71) Applicant: Kissei Pharmaceutical Co., Ltd., Matsumoto-shi Nagano 399-8710 (JP)
(72) Inventor: AKAHANE, Masuo, Matsumoto-shi, Nagano 390-02 (JP); YAMAZAKI, Yoshinobu, Matsumoto-shi, Nagano 390 (JP)
(74) Representative: Baverstock, Michael George Douglas
(86) International application number: JP9702798
(87) International publication number: WO9807445

(57) **Abstract**

The present invention relates to a novel drug for the prevention or treatment of pollakiuria and urinary incontinence which comprises as an active ingredient a β₃-adrenoceptor stimulating agent, which exerts potent relaxing effects on human bladder smooth muscle.

## Description

### Technical Field

The present invention relates to a drug for the prevention or treatment of pollakiuria and urinary incontinence which comprises as an active ingredient a β₃-adrenoceptor stimulating agent.

### Background Art

At present, anticholinergic agents have been mainly used for pollakiuria and urinary incontinence which are diseases related to bladder function. However, it has been shown that anticholinergic agents have a problem of side effects such as dry mouth, constipation and mydriasis. Moreover, it has been reported that they are unsatisfactory in effect for the treatment of pollakiuria and urinary incontinence of the aged. Clenbuterol, a β₂-adrenoceptor stimulating agent, has been used for stress incontinence. On the other hand, it has not been reported that clenbuterol is effective in pollakiuria and age incontinence. Thus, agents hitherto used for the treatment of pollakiuria and urinary incontinence are not satisfactory clinically. Therefore, drugs of a different type which act effectively on bladder smooth muscle are highly desirable.

Accordingly, to develop drugs which act more effectively on human bladder smooth muscle, earlier elucidation of the distribution of some receptors in human bladder smooth muscle has been expected.

It is known that there are three subtypes of β-adrenoceptor, which have been classified as β₁, β₂ and β₃. Each β-adrenoceptor subtype is distributed in specific organs. For example, β₁-adrenoceptor is mainly present in the heart and its stimulation enhances the function of the heart. β₂-Adrenoceptor is mainly present in the trachea, peripheral blood vessels and the uterus, and the smooth muscle of these organs is relaxed by the stimulation of this receptor. Recently, it has been reported that β₃-adrenoceptor is present in the digestive tract, adipocytes and so on. The stimulation of β₃-adrenoceptor leads to the relaxation of gastrointestinal smooth muscle, lipolysis and energy expenditure in adypose tissues.

In general, the distribution of each receptor subtype in organs or tissues is determined by the kind of the species, and there is a difference between each species. For example, it has been reported that β₂-adrenoceptor is dominant as β-adrenoceptor in the bladder of rats and rabbits (The Autonomic Nervous System, Vol.26, pp.380-387 (1989); The Journal of Urology, Vol.139, pp.844-848 (1988); etc.). Up to the present, it has been thought similarly that β₂-adrenoceptor is mainly present in the human bladder (The Journal of Urology, Vol.139, pp.844-848 (1988)).

### Disclosure of Invention

The present invention relates to a drug for the prevention or treatment of pollakiuria and urinary incontinence which comprises as an active ingredient a β₃-adrenoceptor stimulating agent.

The present invention relates to a method for the prevention or treatment of pollakiuria and urinary incontinence which comprises administering a β₃-adrenoceptor stimulating agent.

The present invention relates to a use of a β₃-adrenoceptor stimulating agent for the manufacture of a drug for the prevention or treatment of pollakiuria and urinary incontinence.

Furthermore, the present invention relates to a use of a β₃-adrenoceptor stimulating agent as a drug for the prevention or treatment of pollakiuria and urinary incontinence.

### Brief Description of the Drawings

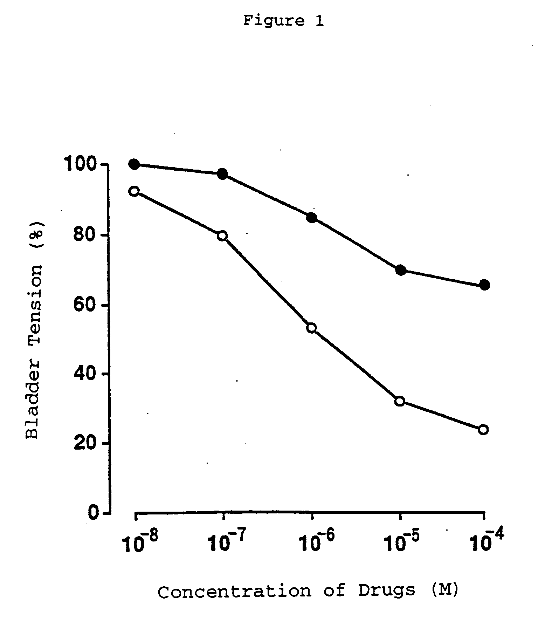

Figure 1 illustrates the effect of each drug on the resting tension of isolated human bladder smooth muscle. The axis of the ordinates shows the bladder tension (%) after drug treatment by each drug. The bladder tension before the treatment is indicated as 100 %, and the tension after the treatment by 10⁻⁵ M of forskolin which produces maximal relaxation of the bladder is indicated as 0 %. The axis of the abscissa shows concentration (M) of used drugs. The symbols -○- and -●- show isoproterenol and CGP-12,177A hydrochloride, respectively.

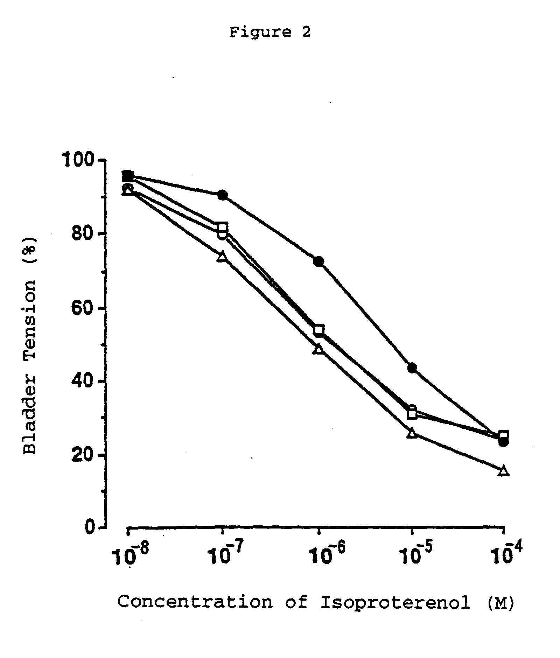

Figure 2 illustrates the relaxing effect of isoproterenol on the resting tension of isolated human bladder smooth muscle and the influence of some β-adrenoceptor blocking agents on the relaxing effect induced by isoproterenol. The axis of the ordinates shows the bladder tension (%) after treatment by each drug. The bladder tension before the drug treatment is indicated as 100 %, and the tension after the treatment by 10⁻⁵ M of forskolin which produces maximal relaxation of the bladder is indicated as 0 %. The axis of the abscissa shows concentration (M) of isoproterenol. The symbols -**○**-, -△-, -□- and -●- show isoproterenol alone, 100 nM metoprolol tartrate + isoproterenol, 100 nM ICI-118,551 hydrochloride + isoproterenol and 1 µM bupranolol + isoproterenol, respectively.

### Best Mode for Carrying Out the Invention

The inventors of the present invention studied extensively drug effects on the human bladder to elucidate the β-adrenoceptor subtypes distributed in the human bladder. As a result, it was found surprisingly that β₃-adrenoceptor is mainly present in human bladder smooth muscle, thereby accomplishing the present invention.

Accordingly, the present inventors carried out the following experiments on the human bladder using isoproterenol as a nonselective β-adrenoceptor stimulant, CGP-12,177A hydrochloride [chemical name: (±)-4-[3-[(1,1-dimethylethyl)-amino]-2-hydroxypropoxy]-1,3-dihydro-2H-benzimidazol-2-on hydrochloride) as a selective β₃-adrenoceptor partial stimulant with β₁- and β₂-adrenoceptors blocking activities (Molecular Pharmacology, Vol.44, pp.1094-1104 (1993)), metoprolol tartrate [chemical name: (±)-1-isopropylamino-3-[p-(β-methoxyethyl)phenoxy]-2-propanol (+)-tartrate] as a selective β₁-adrenoceptor blocker, ICI-118,551 hydrochloride (chemical name: (±)-1-[(2,3-dihydro-7-methyl-1H-inden-4-yl)-oxy]-3-[(1-methylethyl)amino]-2-butanol hydrochloride] as a selective β₂-adrenoceptor blocker and bupranolol [chemical name: 1-(2-chloro-5-methylphenoxy)-3-[(1,1-dimethylethyl)-amino]-2-propanol] as a nonselective β-adrenoceptor blocker and discovered the new fact that β₃-adrenoceptor is dominant in human bladder smooth muscle.

The effects of these drugs on the resting tension of human bladder smooth muscle were investigated. It was found that CGP-12,177A hydrochloride, a selective β₃-adrenoceptor partial stimulant, produced apparent relaxation of bladder smooth muscle. This result indicates that β₃-adrenoceptor is present in the human bladder.

Furthermore, the effects of isoproterenol alone and isoproterenol combined with some β-adrenoceptor blockers were compared in a similar experiment using human bladder smooth muscle. It was confirmed that the relaxing effect of isoproterenol on bladder smooth muscle is not blocked at all by metoprolol tartrate, a selective β₁-adrenoceptor blocker, or ICI-118,551 hydrochloride, a selective β₂-adrenoceptor blocker. However, it was confirmed that the above relaxing effect is blocked by bupranolol, a nonselective β-adrenoceptor blocker. This result indicates clearly that β₃-adrenoceptor is mainly present in the human bladder.

Thus, it was confirmed that β₃-adrenoceptor is mainly present in the human bladder. Accordingly, it was found that drugs having a stimulating effect on β₃-adrenoceptor can provide clinically satisfactory relaxation of the bladder, which cannot be attained using the drugs hitherto used, and are extremely useful as a new type of agent for the prevention or treatment of bladder diseases such as pollakiuria and urinary incontinence.

The present invention relates to a drug for the prevention or treatment of pollakiuria and urinary incontinence which comprises as an active ingredient a β₃-adrenoceptor stimulating agent at least. In the present invention, for example, age incontinence and stress incontinence can be illustrated as urinary incontinence.

The drugs having stimulating effects on β₃-adrenoceptor of the present invention have efficient relaxing effects on the bladder and enhance accumulation of urine by enlarging bladder volume. Therefore, specially, the drugs are effective in pollakiuria and urinary incontinence associated with bladder functional disease. Preferably, in order to act more efficiently and effectively, drugs having highly selective stimulating effects on β₃-adrenoceptor are desirable. Furthermore, drugs having high organ specificity for the bladder or having high affinity for β₃-adrenoceptor in the bladder are more preferred as agents for the prevention and treatment of pollakiuria and urinary incontinence because β₃-adrenoceptor is also present in the digestive tract and adipocytes. In addition, it has been reported that β₂-adrenoceptor is present in the external urethrosphincter and that the stimulation of this receptor leads to the contraction of the external urethrosphincter and is closely related to keeping function in urine accumulation. Therefore, drugs having stimulating effects on both β₂- and β₃-adrenoceptors are more effective in stress incontinence associated with external urethrosphincter functional disease.

The EC₅₀ values for stimulating β₃-adrenoceptor can be measured by investigating the relaxing effects on ferret bladder smooth muscle according to the following method. For example, the EC₅₀ value of CGP-12,177A hydrochloride was 8.1 X 10⁻⁸ (M).

Drugs having markedly weakened β₁-adrenoceptor stimulating effects (stimulating effects on heart function) are preferred in order to reduce burdens on the heart and so as not to induce side effects such as tachycardia.

### EXAMPLES

The contents of the present invention are described further in detail with reference to the following Examples. However, the present invention is not limited thereto.

### Measurement of the contractile force of human bladder smooth muscle strips

### (1) Smooth muscle preparations

Human bladders were obtained from patients undergoing cystectomy and were carefully dissected free from the surrounding fat and mucosa. Preparations were prepared by cutting the bladder smooth muscle longitudinally into about 1.5 cm in length and about 3 mm in width and used.

### (2) Experimental conditions

Buffer solution; the Krebs-Henseleit solution: NaCl (118 mM), KCl (4.7 mM), CaCl₂ (2.5 mM), NaHCO₃ (25.0 mM), MgSO₄ (1.2 mM), KH₂PO₄ (1.2 mM) and glucose (11.1 mM)

An initial tension of about 1 g was set and the effects of drugs on the resting tension were evaluated.

Conditions for measurement; The bathing solution was maintained at 37 °C and gassed with a mixture of 95 % O₂ and 5 % CO₂.

Drug Treatment; The drug was cumulatively added about every 5 minutes.

Evaluation of the drug effects; The drug-induced relaxation was evaluated. The tension before the drug treatment is indicated as 100 %, and the tension after treatment with 10⁻⁵ M forskolin which produces maximal bladder relaxation is indicated as 0 %.

### Example 1

According to the method described above, the bladder relaxing effects of the following drugs were measured using human bladder smooth muscle.

### Drugs used: 1. isoproterenol and 2. CGP-12,177A hydrochloride

The results are indicated in Figure 1.: CGP-12,177A hydrochloride, a selective β₃-adrenoceptor partial stimulant, as well as isoproterenol, a non-selective β-adrenoceptor stimulant, also showed apparent relaxing effects on bladder smooth muscle. These results indicate that the β₃-adrenoceptor is concerned in the relaxation of human bladder smooth muscle.

### Example 2

According to the method described above, the interaction between a β-adrenoceptor stimulant and a β-adrenoceptor blocker was evaluated in human bladder smooth muscle using the following drugs.

### Drugs used: 1. isoproterenol, 2. metoprolol tartrate, 3. ICI-118,551 hydrochloride and 4. bupranolol

The results are indicated in Figure 2.: The pre-treatment with metoprolol tartrate (100 nM), a selective β₁-adrenoceptor blocker, or ICI-118,551 hydrochloride (100 nM), a selective β₂-adrenoceptor blocker, did not attenuate the isoproterenol-induced bladder relaxation at all. On the other hand, the isoproterenol-induced bladder relaxation was apparently weakened by the pre-treatment of bupranolol (1 µM), a non-selective β-adrenoceptor blocker. To judge from these findings, it is also demonstrated that the relaxation of human bladder smooth muscle is hardly mediated via β₁- and β₂-adrenoceptors but is mainly mediated via β₃-adrenoceptor.

### Example 3

### Experiment for measuring the β₃-adrenoceptor stimulating effect (The effects of the drugs on the resting tension in the isolated bladders of ferrets)

The bladders of ferrets were isolated and bladder smooth muscle preparations (about 10 mm in length and about 2 mm in width) were prepared. The experiment was conducted according to the Magnus method. The preparations with a tension of 1 g were suspended in the Krebs-Henseleit solution maintained at 37 °C and gassed with a mixture of 95 % O₂ and 5 % CO₂. The resting tension of bladder was introduced isometrically via a pressure transducer and recorded on a rectigraph. The drug was added cumulatively to the Magnus bath about every 5 minutes. The drug efficacy of the drug was evaluated as 50 % relaxing concentration (i.e., EC₅₀ value). The tension before the drug treatment was indicated as 100 %, and the tension after the treatment by 10⁻⁵ M of forskolin which produces maximal relaxation of the bladder is indicated as 0 %.

### Example 4

### Experiment for measuring the β₂-adrenoceptor stimulating effect (The effects of the drugs on the spontaneous contractions of the isolated pregnant rat myometrium)

The uteri of pregnant SD rats (pregnancy day 21) were isolated and longitudinal preparations (about 15 mm in length and about 5 mm in width) free from the basal plate were prepared. The experiment was conducted according to the Magnus method. The preparations with a tension of 1 g were suspended in the Locke-Ringer solution maintained at 37 °C and gassed with a mixture of 95 % O₂ and 5 % CO₂. The spontaneous contraction of the uterus was introduced isometrically via a pressure transducer and recorded on a rectigraph. The drug was added cumulatively to the Magnus bath every 5 minutes. The drug efficacy was evaluated as 50 % inhibitory drug concentration (i.e., EC₅₀ value) by comparing the sum of uterine contractions during 5 minutes before the addition of the drug with the that during 5 minutes after the addition of the drug.

### Industrial Applicability

A drug comprising as an active ingredient a β₃-adrenoceptor stimulating agent of the present invention exerts potent relaxing effects on human bladder smooth muscle, and is suitable as a drug for the prevention or treatment of pollakiuria and urinary incontinence.

## Claims

1. A drug for the prevention or treatment of pollakiuria and urinary incontinence which comprises as an active ingredient a β₃-adrenoceptor stimulating agent.

2. A drug which comprises as an active ingredient a selective β₃-adrenoceptor stimulating agent as claimed in claim 1 wherein the diseases to be treated are pollakiuria and urinary incontinence associated with bladder functional disease.

3. A drug which comprises as an active ingredient an agent having stimulating effects on both β₂- and β₃-adrenoceptor as claimed in claim 1 wherein the diseases to be treated are pollakiuria and urinary incontinence associated with external urethrosphincter functional disease.

4. A method for the prevention or treatment of pollakiuria and urinary incontinence which comprises administering an effective amount of a β₃-adrenoceptor stimulating agent.

5. A use of a β₃-adrenoceptor stimulating agent for the manufacture of a drug for the prevention or treatment of pollakiuria and urinary incontinence.

6. A use of a β₃-adrenoceptor stimulating agent as a drug for the prevention or treatment of pollakiuria and urinary incontinence.
